# EUROPEAN PATENT APPLICATION

(11) **EP 3 269 732 A1**
(43) Date of publication of application: **17.01.2018**
(21) Application number: 16178903.7
(22) Date of filing: 11.07.2016
(51) Int. Cl.: C07K 14/705, C12N 15/113

(54) **THERAPEUTIC MEDIUM FOR THE TREATMENT OF CANCER**

(71) Applicant: Rhemastem SRLS, 20900 Monza (IT)
(72) Inventor: ZENI, Daniele, 20010 Vanzago (IT); OKERE, Joyce Okechi, 20864 Agrate Brianza (IT)
(74) Representative: Candito, Rocco

(57) **Abstract**

A therapeutic medium comprises an inhibitor of expression and/or activity of MAL2 gene for use in the treatment of cancer, in particular a colon cancer. The therapeutic medium can be made in the form of a pharmaceutical composition, a lentiviral targeting vector or a liposome-nucleic acid particle.

## Description

### BACKGROUND OF THE INVENTION

The present invention relates to a therapeutic medium for treating malignant tumours affecting the human digestive system and, in particular, a therapeutic medium that can be used for treating a colorectal cancer.

### STATE OF THE ART

Cancers of the digestive system are very common and often fatal. For example, colon cancer (colon carcinoma) is the second leading cause of cancer death (Jemal, A. et al. "Cancer statistics" (2006), CA Cancer J Clin 56, 106-30*).* Colon cancer is a disease characterized by uncontrolled cell growth in the tissues of the colon. If left untreated, this cell growth can spread beyond the colon in a process called metastasis into nearby tissue and, eventually, into other parts of the body. Most cancers that start in colon, known as primary colon cancers, are carcinomas that derive from epithelial cells. An increasing number of evidence supports the contention that epithelial cancers are driven by a small sub-population of self-renewing, multipotent cells termed "cancer stem cells" (CSCs) or "cancer-initiating cells" (Jordan C.T., Guzman M.L., Noble M. (2006) "Cancer stem cell" N Engl J Med 355:1253-1261). These cells are also thought to initiate tumour metastasis and relapse after therapy (Jordan C.T., Guzman M.L., Noble M. (2006) Cancer stem cells. N Engl J Med 355:1253-1261 and Bonnet D., Dick J.E. (1997) "Human acute myeloid leukemia is organized as a hierarchy that originates from a primitive hematopoietic cell" Nat Med 3:730-737*).* These cells, which were initially identified in hematopoietic tumours, have been now found in a variety of solid tumours, including breast, brain, pancreas, skin, head and neck, prostate as well as colon cancers (Clay M.R., Tabor M., Owen J.H., Carey T.E., Bradford C.R., Wolf G.T., Wicha M.S., Prince M.E.: "Single-marker identification of head and neck squamous cell carcinoma cancer stem cells with aldehyde dehydro-genase" Head & Neck 2010, 9999*;* and Vermeulen L., De Sousa E., Melo F., van der Heijden M., Cameron K., de Jong J.H., Borovski T., Tuynman J.B., Todaro M., Merz C., Rodermond H., et al.(2010) "Wnt activity defines colon cancer stem cells and is regulated by the microenvironment" Nat Cell Biol, 12:468-476). Colorectal cancer stem cells (CCSCs) were initially isolated as enriched CD133+ cells (Ricci-Vitiani, et al. (2007) "Identification and expansion of human colon cancer initiating cells" Nature 445:111-115*).* This approach has been recently broadened to encompass additional putative markers, including ALDH (aldehyde dehydrogenase), Lrg5, CD166, CD44 and nuclear-β-catenin (Lombardo Y., et al. (2011) "Bone morphogenetic protein 4 induces differentiation of colorectal cancer stem cells and increases their response to chemotherapy in mice" Gastroenterology 140:297-309*;* and Huang E.H., Hynes M.J., Zhang T., Ginestier C., Dontu G., Appelman H., Fields J.Z., Wicha M.S., Boman B.M. (2009) "Aldehyde dehydrogenase 1 is a marker for normal and malignant human colonic stem cells (SC) and tracks SC overpopulation during colon tumorigenesis" Cancer Res 69:3382-3389). The concept that not all of the cells in a tumour possess true tumourigenic ability, down to the most critical capacity to possess typical stem cell-like features and to generate a true copy of the original neoplasia at the single cell level, is the central notion in the now broadening concept that many solid tumours may, in fact, be established, perpetuated and recur after therapy because they contain a relatively rare sub-population of tumour-initiating cells. These are most commonly called "cancer stem cells" (CSCs) or tumour-propagating cells and the existence thereof has been reported in colorectal human cancers, in which relatively rare subsets of CD133+ cells or CD133+/CD44+ cells have been isolated. These subsets of cells were shown to establish colorectal cancer upon heterotopic (subcutaneous injection or subcapsular renal injection) transplantation in mice (Ricci-Vitiani, et al. (2007) "Identification and expansion of human colon cancer initiating cells" Nature 445:111-115*;* and O'Brien C.A., PolletA., Gallinger S. and Dick J.E. (2007) "A human colon cancer cell capable of initiating tumour growth in immunodeficient mice" Nature 445:106-110)*.* An advantageous feature of the CCSCs is their capacity to be proliferated as tumour spheres *in vitro* by means of a serum-free medium in which a combination of epidermal growth factor (EGF) and basic fibroblast growth factor (bFGF or FGF-2) are required to provide mitogenic stimulation. A lethal characteristic of nearly all solid tumours is the ability to generate metastasis. Metastasis is a complex process, which is caused by elaborate interactions between tumour cells and the surrounding normal tissues in different vital organs and accounts for 90 percent of all cancer deaths in patients with solid tumours. A series of models have been used to study the establishment of metastasis, also in colon cancer research. These models include the xenotransplantation of pre-established human cancer cell lines in immunodeficient mice as well as genetic, spontaneous mouse metastatic tumour models. While unquestionably useful, such systems are somewhat prone to loss of consistency in mimicking the original disease, due to spontaneous genetic drift in human cancer cells selected to grow in the presence of bovine serum and due to issues pertaining to the inherent differences that occur between rodent and human tumour cells (Hermann P.C., et al. (2007) "Distinct populations of cancer stem cells determine tumor growth and metastatic activity in human pancreatic cancer", Cell Stem Cell 1:313-323*;* and Chiang A.C., Massagué J. (2008) "Molecular basis of metastasis" N Engl J Med 359: 2814-2823*).* Due to their ability to generate true phenocopies of the human disease over time, these CCSCs represent one of the most suitable models for studying colon cancer physiology in vitro and in vivo and might enable the discovery of genes and/or pathways directly involved in the regulation of the invasive behaviour of the colon cancer itself. Molecular markers that either are found specifically on tumour cells or are highly over-expressed on malignant cells and nearly absent or downregulated on normal cells are attractive therapeutic targets for approaches such as targeted drug delivery.

As previously pointed out, a colon carcinoma is often fatal, especially when it has metastasized, with a median survival time of 6 to 12 months. The vast majority of patients die by two years from diagnosis. There is essentially no cure, and management therapy is commonly based on the combination of surgery, radiotherapy and chemotherapy. However, a drawback of surgery is that the surgical resection of a colon cancer may cause an undesired side effect, i.e. the extensive dissemination of tumour cells within the colon, which often results in tumour recurrence. As for chemotherapy, a drawback of this kind of therapeutic treatment is that it is rather toxic and thus not free from side effects. As a further drawback, chemotherapy is a substantially non-specific treatment, since it targets the overall tumour mass. Similar drawbacks affect the use of radiotherapy. Survival rates have changed very little in over thirty years, which has prompted the active search for new treatments such as gene therapy, anti-angiogenesis, immunotherapy and small molecule transduction inhibitors.

In brief, a strong need is felt of a novel therapeutic medium, such as for example a pharmaceutical composition, which can be used for treating a cancer, in particular a colon cancer, and enables the drawbacks affecting the known therapeutic treatments (chemotherapy; radiotherapy; surgery) to be overcome.

### OBJECTS OF THE INVENTION

An object of the invention is to improve known therapeutic media for treating a cancer.

Another object is to make available a therapeutic medium that is able to effectively inhibit the growth, proliferation and metastasis of a cancer, in particular a cancer affecting the gastrointestinal tract, such as a colon cancer.

A further object is to make available a therapeutic medium for treating a cancer, in particular a colon cancer, which is substantially more specific than known chemotherapeutic compositions.

Another further object is to make available a therapeutic medium for treating a cancer, in particular a colon cancer, which is substantially non-cytotoxic and thus substantially free from side effects.

A further other object is to make available a therapeutic medium for treating a cancer, in particular a colon cancer, which enables to avoid the recourse to surgical resection and/or radiotherapy, as well as the corresponding side effects.

### BRIEF DESCRIPTION OF THE INVENTION

According to the invention, it is provided a therapeutic medium comprising an inhibitor of expression and/or activity of MAL2 gene for use in the treatment of cancer. The therapeutic medium according to the invention is particularly suitable for treating a colon cancer.

The therapeutic medium according to the invention can be - i.e. it can be made in the form of - a pharmaceutical composition, a lentiviral targeting vector or a liposome-nucleic acid particle. Therefore, as used herein, the wording "therapeutic medium" encompasses a pharmaceutical composition, a lentiviral targeting vector or a liposome-nucleic acid particle. In each of these forms of the therapeutic medium, the active ingredient - or active principle - of the therapeutic medium is an inhibitor of expression and/or activity of MAL2 gene. The inhibition of the expression and/or activity of MAL2 gene, which inhibition has to be intended as a down-regulation of the expression and/or activity of the aforesaid gene, is advantageous for blocking tumour progression and metastasis, since it enables the growth of the colon tumour and the cancer stem cell proliferation to be inhibited.

Owing to the invention, the cancer stem cell migration and proliferation can be blocked by inhibiting the MAL2 gene. In particular, it is possible to target a specific cancer stem cell population inside a solid tumour and tailor a therapeutic treatment to deplete the cancer stem cell pool and tumourigenicity. Consequently, a therapeutic medium (such as e.g. a pharmaceutical composition) can be made available that is able to act upon the molecular target of colon cancer, so as to avoid the need of a surgical resection and the consequent possible dissemination of tumour cells within the colon. The therapeutic medium according to the invention has the advantage of acting specifically on the molecular target of colon cancer, thus inhibiting the malignant stem cell cancer progression. Moreover, the therapeutic medium according to the present invention is less toxic and more effective and specific than the commonly used (both alone or combined) radiotherapy and chemotherapy, since the true tumour-initiating cells, rather than the overall tumour mass, are targeted.

Therefore, owing to the invention, a novel therapeutic approach is made available, in which one attempts to "reprogram" the tumour-initiating cell, so as to induce the latter to acquire a non-pathogenic phenotype, rather than trying to kill the tumour cells by cytotoxic means. Further features and advantages will result from the dependent claims and the description.

As used herein, the wordings "MAL2", "MAL-2", "MAL2 gene", "MAL-2 gene", "Mal2", "Mal-2", "Mal2 gene" and "Mal-2 gene" are synonyms and thus can be used interchangeably. As used herein, "inhibitor of expression" and "inhibition of expression" of MAL2, mean respectively a down-regulator and a down-regulation of the expression and/or of the activity of MAL2. As understood by the person skilled in the art, the expression levels of a gene can be quantified by measuring the messenger RNA levels of the aforesaid gene or of the protein encoded by the aforesaid gene. As used herein, the wordings "digestive", "gastrointestinal", "gastroenteric" and "gastroenterological" are synonyms and thus can be used interchangeably. Likewise, as used herein, the wordings "system", "apparatus" and "tract" are synonyms and thus can be used interchangeably. As used herein, "cancer" is defined as an uncontrolled growth of abnormal cells. Such an uncontrolled growth is caused by abnormal and malignant cancer genomes, which are more complex than the genomes of normal cells. As used herein, the wordings "human normal colon cell"/"HNCC" and "human normal colon cells"/"HNCCs" are synonyms and thus can be used interchangeably. Likewise, as used herein, the wordings "colon cancer stem cell"/"CCSC" and "colon cancer stem cells"/"CCSCs" are synonyms and thus can be used interchangeably. As used herein, "metastasis" is defined as the propagation of a cancer from the organ where it started to a different organ. It generally occurs through the blood or lymphatic system. When the cancer cells spread and form a new tumour, the latter is called a secondary or metastatic tumour. The cancer cells forming the secondary tumour are analogous to the cells of the original tumour. If a colon cancer, for example, spreads - i.e. metastasizes - to the liver, the secondary tumour is formed of malignant colon cancer cells. On an anatomical and physiopathological point of view, the alimentary canal and the correlated digestive glands must be regarded as an integrated system, which is responsible for the ingestion, digestion and absorption of food. The aforesaid integrated system includes: mouth, tongue, pharynx, oesophagus, stomach, small intestine (comprising duodenum, jejunum and ileum), large intestine (comprising colon, caecum and rectum), liver, gallbladder, pancreas, spleen. Therefore, in the context of the present invention - and thus in the description and the claims - the cancer to be assayed is at least one of the following: cancer of the mouth, cancer of the oesophagus, cancer of the stomach, cancer of the small intestine (including cancer of duodenum, cancer of jejunum and cancer of ileum), cancer of the large intestine (including cancer of colon, cancer of caecum and cancer of rectum), cancer of the liver, cancer of the gallbladder, cancer of the pancreas, cancer of the spleen. In particular, colon and colorectal cancers are preferably assayed. In the context of the present invention - and thus in the description and the claims - tissues and samples will generally be referred to herein as being from the colon, but it will be appreciated that reference to the colon includes reference to any other part(s) of the digestive system or gastrointestinal tract.

### BRIEF DESCRIPTION OF THE DRAWINGS

The features of the invention can be better understood and implemented with reference to the attached drawings that illustrate an embodiment thereof by way of nonlimiting example, in which:
Figure 1 is a graph showing the relative mRNA expression levels of MAL2 in human Colon-CSC cells analysed by RT-PCR;
Figure 2 is a graph showing the relative mRNA expression levels after down regulation of MAL2 in human colon-CSC cells analysed by RT-PCR;
Figures 3, 4 and 5 are optical microphotographs of isolated colon cancer stem cells (CCSCs), growing as non-adherent spheres in a serum-free medium, comprising a combination of epidermal growth factor (EGF) and basic fibroblast growth factor (bFGF) in order to provide mitogenic stimulation;
Figures 6, 7, 8 and 9 are optical microphotographs of human normal colon stem cells (HNCSCs) growing as non-adherent spheres in a serum-free medium as the one of Figures 3, 4 and 5;
Figure 10 is an optical microphotograph of a normal chromosome metaphase spread, as obtained from a human normal colon stem cell (HNCSC);
Figure 11 is an optical microphotograph of an abnormal chromosome metaphase spread, as obtained from a colon cancer stem cell (CCSC);
Figure 12 is an optical microphotograph showing a representative morphological analysis of HNCSC spheres differentiation following exposure to 1% serum on Matrigel for 5 days.
Figure 13 is an optical microphotograph showing a representative morphological analysis of CCSC spheres differentiation following exposure to 1% serum on Matrigel for 5days.
Figure 14 is a graph showing the results (data are mean ± s.d. of three different experiments in duplicate) of a quantitative RT-PCR (qRT-PCR) analysis of mRNA expression levels of putative stem cell marker genes ALDH1a, BMI 1, LGR5, OLFM-4 and CD133 in CCSC and HNCSC spheres;
Figure 15 is a graph showing the growth kinetics curves of 3 different colon cancer stem cells lines at early and late passages before the down-regulation of MAL2;
Figure 16 is a graph showing the growth kinetics curves of human normal colon stem cells in culture;
Figure 17 is a graph showing the growth kinetics curves after down-regulation of MAL2 in colon cancer stem cells in culture;
Figure 18 is a graph showing the results of serial clonogenic assays in colon cancer stem cells;
Figure 19 is a graph showing the results (mean ± s.e.m.) of an *in vivo* detection of differences in tumour growth and tumour size following subcutaneous injection of luc-CCSCs and luc-CCSCs-MAL2, at 30 days after subcutaneous injection, the aforesaid detection consisting in measurements of subcutaneous tumour bioluminescence in 3 groups of 10 mice;
Figure 20 is a photograph showing an *ex vivo* BLI (Bioluminescence Imaging) image of a subcutaneous tumour of control experimentally induced by a subcutaneous injection of luc-CCSCs;
Figure 21 is a photograph showing an *ex vivo* BLI (Bioluminescence Imaging) image of a subcutaneous tumour of control experimentally induced by a subcutaneous injection of luc-CCSCs-MAL2;
Figure 22 is a photograph showing a comparison between the subcutaneous tumour of Figure 20 and the subcutaneous tumour of Figure 21;
Figure 23 is a series of photographs showing an *in vivo* BLI (Bioluminescence Imaging) monitoring of tumour progression and metastatic dissemination after transplantation of luc-CCSCs;
Figure 24 is a series of photographs showing an *in vivo* BLI (Bioluminescence Imaging) monitoring tumour progression and metastatic dissemination after transplantation of luc-CCSCs-MAL2;
Figure 25 is a broken line graph showing the growth rate of primary tumours and regional metastasis as quantified weekly by bioluminescent intensity;
Figure 26 is a histogram graph showing the growth rate of primary tumours and regional metastasis as quantified weekly by bioluminescent intensity;
Figure 27 is a photograph of an *in vivo* ventral view of a representative mouse transplanted with luc-CCSCs, showing a development of lymph node and lung metastasis;
Figures 28 and 29 are two optical microphotographs of corresponding H.E. (haematoxylin and eosin) stained histological sections showing local primary tumour formation at the site of implantation (colon);
Figure 30 is an optical microphotograph of a H.E. stained histological section showing the site of implantation of the luc-CCSCs-MAL2 without tumour development;
Figure 31 is an optical microphotograph of a H.E. stained histological section of mouse pancreas showing metastatic tumours derived from luc-CCSCs at 42 days after orthotopic implantation;
Figure 32 is an optical microphotograph of a histological section as the one of Figure 31, wherein the histological section was obtained from mouse liver;
Figure 33 is an optical microphotograph of a histological section as the one of Figure 31, wherein the histological section was obtained from mouse kidney;
Figure 34 is an optical microphotograph of a histological section as the one of Figure 31, wherein the histological section was obtained from mouse lymph node;
Figure 35 is an optical microphotograph of a histological section as the one of Figure 31, wherein the histological section was obtained from mouse lung;
Figure 36 is an optical microphotograph of a histological section of mouse colon, wherein avidin-biotin-peroxidase complex was used and CDX2 immunohistochemical marker reveal comparable staining patterns in primary and secondary metastatic xenografts;
Figure 37 is an optical microphotograph as the one in Figure 36, wherein the histological section was obtained from mouse kidney;
Figure 38 is an optical microphotograph as the one in Figure 36, wherein the histological section was obtained from mouse pancreas;
Figure 39 is an optical microphotograph as the one in Figure 36, wherein the histological section was obtained from mouse liver;
Figure 40 is an optical microphotograph as the one in Figure 36, wherein the histological section was obtained from mouse muscle;
Figure 41 is an optical microphotograph as the one in Figure 36, wherein the histological section was obtained from mouse small intestine;
Figure 42 is an optical microphotograph as the one in Figure 36, wherein the histological section was obtained from mouse kidney;
Figure 43 is an optical microphotograph of a histological section of mouse pancreas, wherein avidin-biotin-peroxidase complex was used and CK20 immunohistochemical marker reveal comparable staining patterns in primary and secondary metastatic xenografts;
Figure 44 is an optical microphotograph as the one in Figure 43, wherein the histological section was obtained from mouse liver;
Figure 45 is an optical microphotograph as the one in Figure 43, wherein the histological section was obtained from mouse kidney;
Figure 46 is a photograph showing a liver excised from a nu/nu mouse orthotopically transplanted with luc-CCSCs, wherein a white spot indicates tumour cells and a black spot indicates normal tissue;
Figure 47 is a graph showing the experimental results of *in vitro* cell growth of primary luc-CCSCs and secondary (i.e. metastatic) luc-CCSs.

### DETAILED DESCRIPTION OF THE INVENTION

The Inventors realized that a suitable model is necessary to study metastasis, in order to understand the molecular and cellular mechanisms that lead primary tumours to form metastasis and thus better address this major life-threatening problem. The identification of genes that can block metastasis, or suppress the latter, is essential for understanding the basic biology of this lethal condition and its implications for clinical practice. Metastasis-mediating genes would eventually constitute worthy diagnostic markers and therapeutic targets. The Inventors focused their attention on the human gene MAL2, the unregulated over-expression of which is correlated with tumourigenesis (tumour growth and survival) and associated metastasis in human cancer progression. MAL2, a novel member of the MAL proteolipid family, was identified from a yeast 2-hybrid screen with TPD52L2 as bait (Wilson S.H.D., Bailey A.M., Nourse C.R., Mattei M. G., Byrne J.A., Genomics 76:81-88(2001*) "Identification of MAL2, a novel member of the mal proteolipid family, though interactions with TPD52-like proteins in the yeast two-hybrid system ")* and a MAL2 cDNA was cloned from a human breast carcinoma cDNA library. MAL2 encodes a deduced 176-amino acid protein with 4 transmembrane domains and a (Q/Y)GWVM(F/Y) sequence found in all known MAL-like sequences. The MAL2 protein shares 35.8% sequence identity with MAL, a proteolipid required in apical vesicle transport, and 34.5% with BENE protein. Northern blot analysis detected a 2.8-kb MAL2 transcript at high levels in kidney, at moderate levels in brain, liver and lung, and at low levels in colon, heart, placenta, and small intestine. A 1.2-kb transcript was also weakly detected in kidney and liver. Both transcripts were detected in human breast carcinoma tumor and cell lines. By Northern blot analysis, the 2.8-kb MAL2 transcript was detected in thyroid, stomach, and testis (Marazuela M., Martin-Belmonte F., Garcia-Lopez M.A., Aranda J.F., de Marco M.C., Alonso M.A. Endocrinology 145:1011-1016(2004*) "Expression and distribution of MAL2, an essential element of the machinery for basolateral-to-apical transcytosis, in human thyroid epithelial cells"*). The sequence of human MAL2 gene is known and published by PubMed (NCBI) with accession number NM_052886.2.

The Inventors devised and developed a biologic model that allows to understand how the metastatic cells carry out their lethal function at various stages. In particular, the Inventors found that the aforesaid MAL2 gene is abnormally expressed in the colon cancer stem cells compartment with regards to the normal control and realized that such a finding could be used for treating and preventing colon cancer and colon cancer metastasis. The aforesaid biological model provides for carrying out the following steps: i) Cultivating the CCSCs that possess all of the features expected from true colon tumour-initiating cells, including the ability to generate metastatic lesions; ii) Using the aforesaid cultivated CCSCs to identify or select therapeutic agents that are effective against cancers, in particular against cancers affecting the digestive system or gastrointestinal tract.

The biological model above represents a novel approach for the study of colon cancer and provides a system in which colon cancer metastatic behaviour is effectively reproduced and replicated, also under standard and reproducible conditions that allow for *in vivo* monitoring of the spreading and growth of human colorectal cancer metastasis. It is undisputed that such a model significantly helps to study cancer and to identify effective therapeutic treatments. In the aforesaid COLON-CSCs-based model, human colon carcinoma xenograft phenocopies are established in the colon wall. In particular, the COLON-CSCs are transplanted into the colon wall of immunodeficient mice (orthotopic transplant) and the tumours consequently established display a typical colon cancer metastatic pattern. In fact, following the establishment of a typical colon cancer lesion at the initial colon injection site (in the immunodeficient mice), the aforesaid cancer lesion not only expanded in size, but eventually gave metastatic lesions in distant organs. This occurred in a progressive manner that saw metastasis spreading to the liver, stomach, kidney pancreas and lymph nodes and, at later stages and in a few animals, to the lungs. In this way, the cancer can be reproduced experimentally by a xenograft system better respecting physiological conditions. Moreover, the fact that the cells (to be transplanted) are easily manipulated enables firefly luciferase tagged CCSCs primary lines to be established. It is thus possible to perform a continuous, quantitative analysis of colon tumour growth *in vivo,* under orthotopic and more suitable conditions, by using known and standardized imaging techniques and starting as early as 3 days after transplantation.

In the following, the cogent results of cell growth kinetic, serial clonogenic analysis *in vitro* and *in vivo* serial transplantation experiments will be disclosed, which results clearly demonstrate the virtually unlimited self-renewal capacity, significant expansion potential of CCSCs deriving from the human colorectal carcinomas. In these cell lines, the tumour-initiating cells that can reproduce the original colorectal cancer unfold their self-renewal potential, so that the CCSCs are demonstrably perpetuated and amplified in number, as shown by their stable molecular, growth, differentiation and tumour-initiating features, over virtually unlimited time. In this way, a system is provided in which the main features of the CCSCs and models of colorectal carcinomas can be stably and faithfully reproduced on a broad scale and over time. A very important finding deriving from the experimental use of the CCSCs-based model is that the cells that establish the metastasis in distant organs are, themselves, true colon cancer stem cells. In fact, some cells of the progeny of the luciferase tagged CCSCs injected in the colon eventually established metastasis in the liver of immunodeficient mice. Various experimental procedures and analyses (serial clonal and sub-clonal culturing and transplantation; serial transplantation analyses), both under heterotopic and orthotopic conditions, revealed that the luciferase-positive cells from the metastasis possessed the same functional features as their colon-injected mother cells. Therefore, the cells that establish metastasis in colorectal cancer are derived from CCSCs and somehow retain the features of true colon CCSCs in the metastatic lesions.

In the therapeutic medium according to the invention, the inhibitor of expression and/or activity of MAL2 gene can be a shRNA. As known to the person skilled in the art, a "small hairpin RNA" or "short hairpin RNA" (shRNA) is an artificial RNA molecule, which is provided with a tight hairpin turn and can be used to silence target gene expression via RNA interference (RNAi). RNAi is a biological process in which RNA molecules inhibit gene expression by causing the destruction of specific mRNA molecules. The expression of shRNA in cells is typically accomplished by molecular vectors, such as plasmids encoding specific shRNA molecules, or through viral or bacterial vectors. When the shRNA is encoded in a plasmid, the latter substantially contains a corresponding DNA transposable sequence (insert sequence), including a DNA template which will be transcripted as shRNA in the target cell. For example, the Inventors tested the use of plasmids (see Example 4, paragraph 4.1) containing DNA templates coding for shRNAs acting upon the following nucleotide target-sequences (sense strands) of the MAL2 gene:
5'-TGGCTCAAATTGATGCTAACT-3' (corresponding to positions 215-235 of MAL2 gene);
5'-TTGGTTGCCTCCTCCAATGTT-3' (corresponding to positions 258-279 of MAL2 gene);
5'-CTCCTGAGTGATAACCAGTAT-3'(corresponding to positions 374-394 of MAL2 gene);
5'-CTTCGTCTGCCTGGAGATTCT-3'(corresponding to positions 519-540 of MAL2 gene).

The aforesaid target-sequences are included in the Sequence Listing (complying with Standard ST.25 WIPO) attached to the present Application, in which Sequence Listing they are respectively named SEQ ID NO: 1, SEQ ID NO: 2, SEQ ID NO: 3, SEQ ID NO: 4. According to the analytical tests (RT-PCR) carried out by Inventors, the down-regulation of MAL2 gene resulted to be particularly effective when using plasmids that were specific for the target-sequence 5'-TTGGTTGCCTCCTCCAATGTT-3' and the target-sequence 5'-CTCCTGAGTGATAACCAGTAT-3'. It should be pointed out that, in addition to the specific examples of shRNA above, every shRNA - and thus every oligonucleotide sequence - that is able to inhibit the expression of MAL2 gene can be suitably used for carrying out the present invention. Therefore, the inhibitors of expression and/or activity of MAL2 gene - which can be incorporated as active principles in the therapeutic medium according to the invention - can act upon target-sequences of the MAL2 gene that comprise, but are not limited to, SEQ ID NO: 1, SEQ ID NO: 2, SEQ ID NO: 3 and SEQ ID NO: 4.

A therapeutic medium according to the invention, which contains the shRNA and/or other DNA sequence(s) suitable for inhibiting (namely, down-regulating) the expression of MAL2 gene, can be - i.e. it can be made in the form of - a pharmaceutical composition, a lentiviral targeting vector or a liposome-nucleic acid particle.

When the therapeutic medium according to the invention is a pharmaceutical composition, in particular a pharmaceutical composition for treating a cancer, it can be prepared according to known techniques and then administered to a human or animal subject. In every instance, a pharmaceutical composition according to the invention will comprise an oligonucleotide that is capable of inhibiting the expression of MAL2 gene. The aforesaid pharmaceutical composition will be sterile, pyrogen/free and will include formulations suitable for human and veterinary use. Suitable methods for preparing the aforesaid pharmaceutical composition are known to the person skilled in the art (see for example: Remington's Pharmaceutical Science, 17th ed., Mack Publishing Company, Easton, Pa., 1985*).* A pharmaceutical composition according to the invention comprises shRNA, e.g. 0.1% to 90% by weight, or a physiologically acceptable salt thereof, in combination with a physiologically and pharmaceutically acceptable carrier. Suitable carriers comprise, but are not limited to, water, buffered water, salt solutions (e.g. normal salt or balanced salt solutions, such as Earle's balanced salt solution or Hanks' balanced salt solution), 0.4% saline, 0.3% glycine, hyaluronic acid and the like. The composition according to the invention can also comprise known pharmaceutical excipients and/or additives. Suitable pharmaceutical excipients comprise, but are not limited to, stabilizers, antioxidants, osmolality adjusting agents, buffers, and pH adjusting agents. Suitable additives comprise, but are not limited to, physiologically biocompatible buffers (e.g., tromethamine hydrochloride), chelating agents (such as e.g. DTPA or DTPA-bisamide), calcium chelate complexes (such as e.g. calcium DTPA, CaNaDTPA-bisamide), or, optionally, calcium or sodium salts (such as e.g. calcium chloride, calcium ascorbate, calcium gluconate or calcium lactate). The pharmaceutical composition according to the invention can be formulated and packaged for use in a liquid form or in a lyophilized form.

The therapeutic medium according to the invention can be a lentiviral targeting vector. In a lentiviral targeting vector according to the invention, a nucleic acid is incorporated that is a shRNA, or any other oligonucleotide, which is capable of inhibiting the expression of MAL2 gene. In particular, the therapeutic medium can be made in the form of a viral vector, which contains a DNA sequence encoding a shRNA and can be used for *in vivo* delivery of the shRNA to target cells. In this way, it is possible to target a particular tissue, a particular class of cell types, a particular cell type or subtype. It is known to the person skilled in the art that a specific lentiviral vector can be generated with a heterologous targeting polypeptide that exhibit binding affinity to a unique cognate binding partner on the targeted tissue, class, cell type or subtype for the delivery of a therapeutic molecule. According to the present invention, the therapeutic molecule comprises a shRNA, or any other oligonucleotide, that is capable of inhibiting the expression of MAL2 gene. The aforesaid inhibition will be achieved through the expression of the shRNA - or of any other suitable oligonucleotide, as incorporated in the viral vector - in the targeted cell or tissue, resulting in the production of a polypeptide having the desired activity (down-regulation of MAL2 gene). A predetermined binding specificity can be given to the vector by incorporating into a vector envelope a targeting polypeptide having a desired binding specificity. The targeting polypeptide can be incorporated through various procedures, such as: normal cellular processes for transmembrane insertion, or for membrane attachment or anchoring, of polypeptides, lipids and other macromolecules (in which case, the targeting polypeptide can contain e.g. a transmembrane domain or a membrane attachment domain that incorporate into, or are capable of incorporating into, a lentiviral vector envelope); noncovalent association of a targeting polypeptide with an envelope associated polypeptide, lipid, carbohydrate or other molecule; chemical conjugation; incorporation (of an *in vitro* or chemically synthetized targeting polypeptide) through a cell-free system; automatic incorporation (into a lentiviral vector envelope) through expression of a recombinant or synthetic nucleic acid encoding the targeting polypeptide in a vector packaging system. From the above, it is clear that the therapeutic medium according to the invention can be made in the form of a lentiviral targeting vector by applying procedures that are known to a person skilled in the art.

The therapeutic medium according to the invention can be a liposome-nucleic acid particle, or lipoplex, or nucleic acid-lipid particle. As used herein, the wordings "liposome-nucleic acid particle(s)", "lipoplex(es)" and "nucleic acid-lipid particle(s)" are to be considered as synonyms and thus can be used interchangeably. In a liposome-nucleic acid particle according to the invention, the nucleic acid is a shRNA, or any other oligonucleotide, that is capable of inhibiting the expression of MAL2 gene. Therefore, as used herein (and thus both in the description and claims), the wording "liposome-nucleic acid particle" defines a liposome-nucleic acid particle, in which the nucleic acid can be a shRNA, or any other oligonucleotide, that is capable of inhibiting the expression of MAL2 gene. Methods for delivering RNAi molecules - such as shRNA molecules - to tumour cells using complexes (lipoplexes) prepared by mixing an RNAi molecule with a liposome are known (Qixin Leng et al., Drug Future, 2009 September, 34(9), 721*;* Sherry Y. Wu et al., The AAPS Journal, Vol. 11, No. 4, December 2009*;* B. Ozpolat et al., Journal of Internal Medicine 267; 44-53 2009*).* For the person skilled in the art, a "lipid particle" is a stable, substantially non-toxic lipid formulation that can be used to deliver an active agent or therapeutic agent to a specific target site, such as a cell, a tissue, an organ, and the like. The lipid particle can be a nucleic acid-lipid particle, which is typically formed from a cationic lipid, a non-cationic lipid, and optionally a conjugated lipid that prevents aggregation of the particle. According to embodiments of the invention, the nucleic acid can be partially or fully encapsulated in the lipid portion of the particle and thus protected from enzymatic degradation, e.g. it can be made resistant to degradation by a nuclease in an aqueous solution. The conjugated lipid, which is able to inhibit aggregation of lipid particles, includes, but is not limited to: PEG-lipid conjugates, such as e.g. PEG coupled to dialkyloxypropyls (e.g. PEG-DAA conjugates), PEG coupled to diacylglycerols (e.g. PEG-DAG conjugates), PEG coupled to cholesterol, PEG coupled to phosphatidylethanolamines, PEG conjugated to ceramides, cationic PEG lipids, polyoxazoline (POZ)-lipid conjugates (e.g. POZ-DAA conjugates; polyamide oligomers (e.g. ATTA-lipid conjugates), and mixtures thereof. PEG or POZ can be conjugated directly to the corresponding lipid or may be linked to the lipid via a linker moiety. Any linker moiety suitable for coupling the PEG or the POZ to a lipid can be used, such as e.g. non-ester containing linker moieties and ester-containing linker moieties. According to an embodiment of the invention, non-ester containing linker moieties, such as amides or carbamates, are used. The lipid particles, in which an active agent or therapeutic agent (e.g. shRNA) is entrapped within the lipid portion of the particle and is protected from degradation, can be prepared by using any method that known to the person skilled in the art. Once prepared, the lipid particles can be administered to a human (or animal) subject either alone or in a mixture with a pharmaceutically acceptable carrier (e.g. physiological saline or phosphate buffer), which is selected in accordance with the route of administration and standard pharmaceutical practice. Generally, normal buffered saline (e.g., 135-150 raM NaCl) can be used as the pharmaceutically acceptable carrier. Other suitable carriers include, but are not limited to, water, buffered water, 0.4% saline, 0.3% glycine, glycoproteins for enhanced stability (e.g. albumin, lipoprotein, globulin). Further pharmaceutically acceptable carriers, which are suitable for carrying out the present invention, are known to the person skilled in the art (see for example: Remington's Pharmaceutical Science, 17th ed., Mack Publishing Company, Easton, Pa., 1985)*.* As used herein, the wording "carrier" includes any and all solvents, dispersion media, vehicles, coatings, diluents, antibacterial and antifungal agents, isotonic and absorption delaying agents, buffers, carrier solutions, suspensions, colloids, and the like. The liposome-nucleic acid particles according to the invention can be administered in a manner that is compatible with the dosage formulation and in such amount as to be therapeutically effective. For example, the liposome-nucleic acid particles can be incorporated in injectable solutions or drug release capsules, the preparation methods of which are well known to the person skilled in the art. If so desired, known supplementary active ingredients can be incorporated into the injectable solutions or drug release capsules, together with liposome-nucleic acid particles and the pharmaceutically acceptable carrier. In addition, the liposome-nucleic acid particles can be administered via a systemic delivery, i.e. the liposome-nucleic acid particles are administered in such a way that they enters a patient's circulatory system. The liposome-nucleic acid particles can be administered by direct injection at the site of disease or by injection at a site distal from the site of disease. In brief, it is clear that the therapeutic medium according to the invention can be made in the form of a liposome-nucleic acid particle (or lipoplex, or nucleic acid-lipid particle) by applying procedures that are known to a person skilled in the art.

With regards to the route of administration of the therapeutic medium according to the invention, it should be pointed out that any suitable route of administration can be used, as long as the selected route is compatible with the form (pharmaceutical composition, lentiviral targeting vector or liposome-nucleic acid particle) of the therapeutic medium that is to be administered and is able to provide a treated subject with an effective dosage. The therapeutic medium according to the invention can thus be administered and dosed in accordance with good medical practice, taking into account the clinical condition of the individual patient, the disease to be treated, the site and method of administration, scheduling of administration, patient age, sex, body weight and other factors that are well known to the person skilled in the art, i.e. medical practitioners. Although a preferred method of delivery is the systemic delivery, in which the therapeutic medium is administered in such a way that it enters a patient's circulatory system, however other delivery methods can be suitably used. For example, the therapeutic medium according to the invention can be administered orally (e.g. by tablets, troches, dispersions, suspensions, solutions, capsules), transdermally (e.g. by patches), subcutaneously or parenterally, including the use of intravenous, intra-arterial, intramuscular, intraperitoneal, intrathecal routes (in which routes liquid forms of the therapeutic medium are used that are suitable for injection) as well as the use of inhalation, intranasal administration, transtymopanic administration, and infusion techniques. Implants of known devices, which are suitable for dispensing the therapeutic medium to the patient's body, can also be used. The therapeutic medium according to the invention can be prepared as a liquid composition, including: aqueous solutions with and without organic co-solvents, aqueous or oil suspensions, emulsions with edible oils, as well as similar pharmaceutical vehicles.

With regards to the type of subject who can be treated by using the therapeutic medium according to the invention, the aforesaid subject includes warm-blooded animals and, in particular, mammals including human. The term "treatment" - and in particular "treatment of cancer" - as used herein, means administering to the subject an amount of an inhibitor which inhibits (i.e. down-regulates) the expression of the MAL2 gene. In some cases, more than one inhibitors can be administered, at the same time or sequentially. Therefore, carrying out a treatment by using the therapeutic medium according to the invention substantially corresponds to administering to the subject a therapeutically effective dose of one or more inhibitors, in particular shRNA, which down-regulate the expression of the MAL2 gene. Treatment regimens may vary and often depend on tumour type, tumour location, disease progression, health and age of the subject. It is clear to the person skilled in the art, namely the clinician, that certain types of tumour will require a more aggressive treatment, while at the same time, certain patients cannot tolerate more taxing protocols. The clinician will be able to make such decisions based on the known efficacy and toxicity (if any) of the formulation(s) of the therapeutic medium according to the invention. It should be noted that, in certain cases, the tumor being treated may not, at least initially, be resectable. However, treatments with therapeutic viral constructs - such as the therapeutic medium made in the form of a lentiviral targeting vector - may increase the surgical resectability of the tumor, owing to shrinkage at the margins or by elimination of certain particularly invasive portions. Following a treatment with the therapeutic medium according to the invention, surgical resection may be possible. Additional treatments subsequent to the surgical resection will serve to suitably eliminate microscopic residual disease at the tumor site. A treatment carried out with use of the therapeutic medium according to the invention may comprise administering a number of unit doses. As used herein, "unit dose" means a predetermined quantity of the therapeutic medium according to the invention. The selection of suitable quantities to be administered, as well as the particular administration route and formulation to be used, are within the knowledge of the person skilled in the art, i.e. a clinician and/or a pharmacologist. The administered dose of the therapeutic medium must be effective to achieve improvement including, but not limited to: improved survival rate, more rapid recovery, improvement of symptoms, elimination of symptoms and/or other suitable clinical indicators as selectable by the person skilled in the art. In general, the therapeutically effective dose (for humans) of the active ingredient - namely an inhibitor of expression and/or activity of MAL2 gene - as incorporated in the therapeutic medium according to the invention is in the range of from 1 ng/kg to about 20-100 mg/kg body weight per day, preferably about 0.01 mg to about 2-10 mg/kg body weight per day, in a regimen of one dose per day or twice or three or more times per day for a period of 1-4 weeks or longer.

The scientific bases of the invention have been experimentally confirmed by the Applicant in the following Examples 1-9. These Examples are included herein for the purpose of enabling the invention to be fully understood, but they are not intended to limit in any manner the scope of the invention.

### EXAMPLE 1 - Cell cultures

### 1.1 Isolation of stem cells from colon cancer and normal mucosa samples

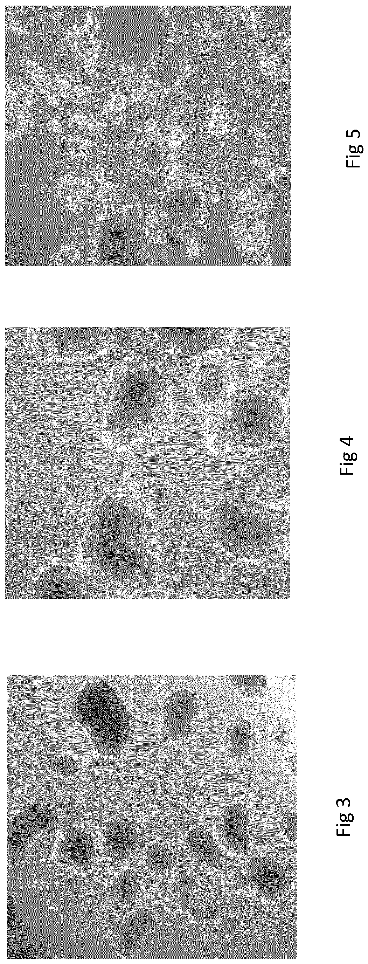

Normal and neoplastic stem cells from neural and epithelial organs can be expanded as sphere-like cellular clusters in a serum-free medium containing EGF and bFGF *(*Singh S.K., et al. (2003) "Identification of a cancer stem cell in human brain tumors" Cancer Res. 63:5821-5828*;* and Dontu G., et al. (2003) "In vitro propagation and transcriptional profiling of human mammary stem/progenitor cells" Genes Dev. 17:1253-1270). Adult human neoplastic and normal mucosa were obtained after surgical resections. The tissues were mechanically and enzymatically disaggregated into single-cell suspensions as previously described (Dalerba P., et al. (2007) "Phenotypic characterization of human colorectal cancer stem cells" PNAS 104:10158-10163*).* Briefly, solid tissues were mechanically dissociated and, resuspended in DMEM (Dulbecco's Modified Eagle Medium) containing 100 µg/ml of Penicillin, 100 µg/ml of Streptomycin, 0.25 µg/ml of Amphotericin B, 50 µg/ml of Gentamicin, together with 200 units/ml of Collagenase type III (Worthington, Lakewood, NJ) and 100 units/ml of DNase I (Worthington), and incubated for 5-6 hrs at 37°C to obtain enzymatic disaggregation. Cells were then resuspended by pipetting and serially filtered by using sterile gauze and 70 µm and 40µm nylon meshes. The cells were collected by centrifugation and single cells were plated in 25cm² tissue culture flasks (Nunc). The resulting cancer cells were cultured in a serum-free medium (StemPro® NSC SFM kit from Lifetech) containing EGF and bFGF, supplemented with 0.4 µg of BSA (Sigma) and a lipid mixture (Sigma), and then grown in a 37°C incubator in a 5% CO₂ and 5% O₂ atmosphere. After 4-6 weeks of culture, colon-spheres were obtained that were formed by clusters of exponentially growing undifferentiated cells. These cell clusters are shown in Figures 3, 4, 5 and Figures 6, 7, 8 and 9. The number of primary spheres, as generated in each flask, was assessed 30-40 days after plating. Spheres were then collected in a 15ml tube (Beckton Dickinson) by centrifugation for 10 min. at 1000 rpm. Primary spheres were then mechanically dissociated to a single-cell suspension and re-plated for culturing. In a number of days which was typical for each cell line, and comprised between 7 and 10 days, new spheres were formed that could undergo further passaging. For the normal tissues, the days were comprised between 10 and 15. The cancer cell lines were identified with the name "colon cancer stem cells" (CCSCs) and the normal cell lines were identified with the name "human normal colon stem cells" (HNCSCs). Part of the CCSCs from successfully established cell lines were frozen down at every sequential third or fourth sub-culturing step in the serum-free medium plus 10% DMSO (dimethyl sulfoxide) (Sigma) and stored, whilst the remainder of the cells was further grown in culture. Karyotyping was performed for all normal cell lines, as it will be described below, and successful lines were frozen down after 20 passages to be used as control for further experiments.

### 1.2 Chromosome analysis

Chromosome karyotyping was performed according to a known standard protocol. The cells were recovered from culture. After centrifugation (1200 rpm for 15 min), the pellets were resuspended in 75 mM KCl solution and incubated for 15mins at 37°C. Cells were centrifuged (1200 rpm for 15 min), fixed in methanol-acetic acid (3:1), air- dried on slides, stained with DAPI (4',6-diamidino-2-phenylindole) and microscopically analyzed. A total of 10 metaphases were counted and analyzed.

### Results

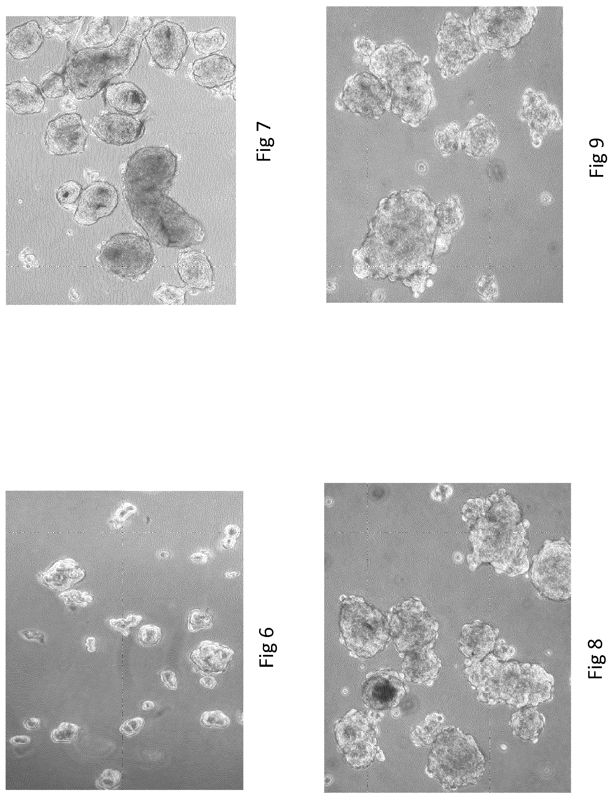

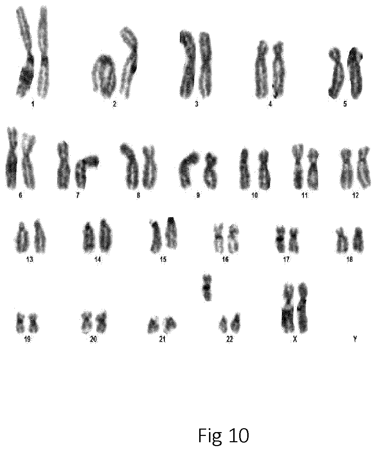

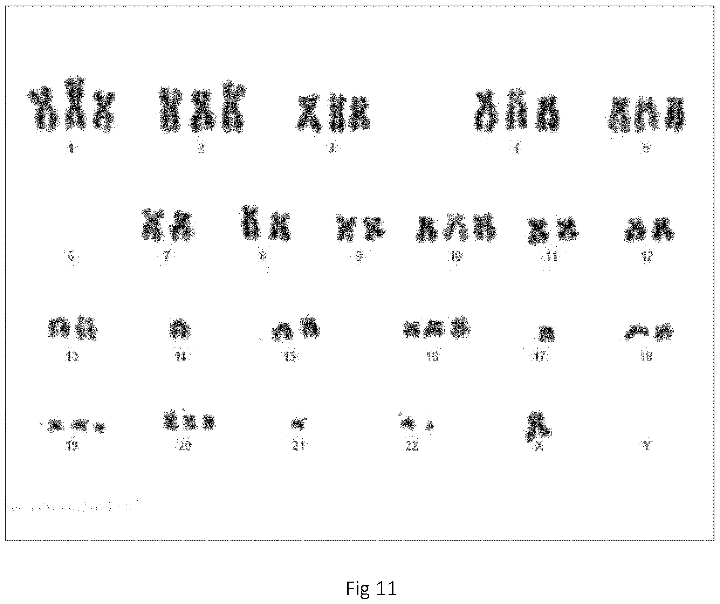

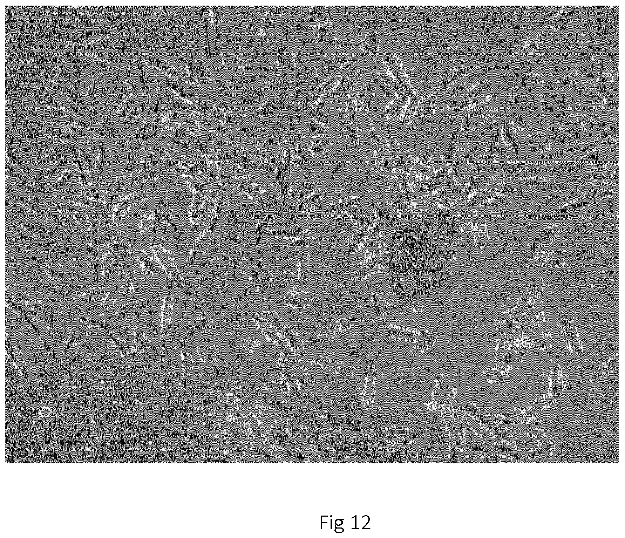

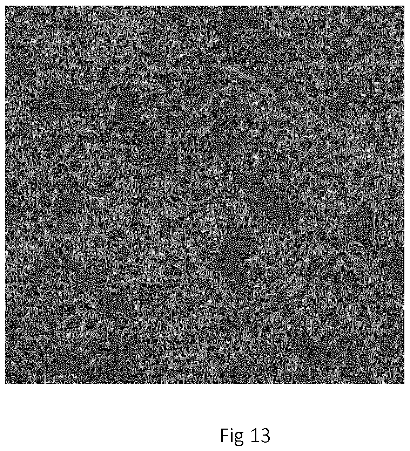

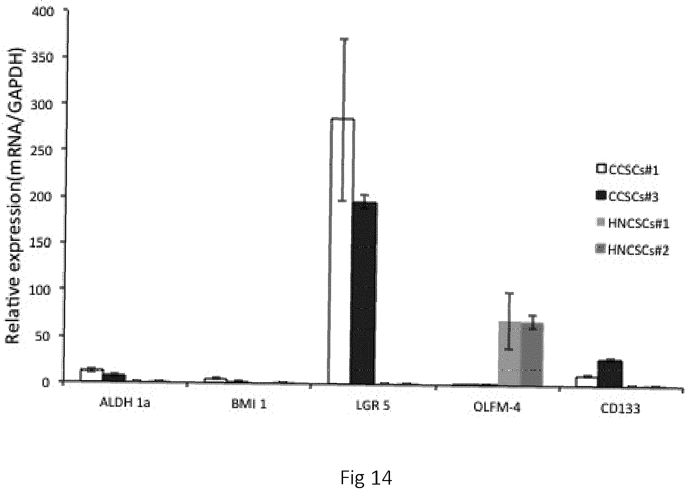

To confirm that the cells in culture possessed the self-renewal ability that is typical of normal and cancerous stem cells, they were grown as spheroids (see Figures 3-5, showing isolated colon cancer stem cells, and Figures 6-9, showing human normal colon stem cells). Moreover, the cells in culture could give rise to secondary spheres and eventually stable cell lines which would retain stable differentiation potential: see Figures 12 and Figure 13, respectively showing a representative morphological analysis of HNCSC spheres differentiation (Figure 12) and CCSC spheres differentiation (Figure 13) following exposure to 1% serum on Matrigel for 5 days. Furthermore, these cells were enriched for putative markers of tumor-initiating cells of colon cancer: see the graph in Figure 14, showing the results (data are mean ± s.d. of three different experiments in duplicate) of a quantitative RT-PCR (qRT-PCR) analysis of mRNA expression levels of putative stem cell marker genes ALDH1a, BMI 1, LGR5, OLFM-4 and CD133 in CCSC and HNCSC spheres. The HNCSCs exhibited a normal chromosome metaphase spread at day 180 (as shown in Figure 10), i.e. a normal karyotype, as compared to the chromosome metaphase spread at day 180 of a CCSC (as shown in Figure 11). The study of regulatory mechanisms of normal colon stem cells may lead to the identification of novel inhibitors of CCSCs and may result in the development and identification of more specific therapeutic strategies for colon cancers.

### EXAMPLE 2 - Gene expression analysis

### 2.1 Microarray analysis

A total of 10 samples (CCSCs nos. 1 to 5 and HNCSCs nos. 6 to 10), taken from established cell lines, were used for a microarray experiment. 10 total RNA samples were transcriptionally profiled using "Affymetrix GeneChip HuGene-2_0-st-v1" arrays. Samples 1-5 were associated to a "Tumour" condition, while samples 6-10 were associated to a "Normal" condition. PCR analysis (which will be described in more detail below) underlined marked differences between "Tumour" cell populations. Analysis results were performed by using commercial software "Partek Genomics Suite" updated to version 6.6. The most updated version of probe sets annotations (containing static information specific to the probe set composition, sequence annotations extracted from public databases and protein sequence-level annotations derived from public domain program) available at the moment of analysis were retrieved by "Affymetrix" website through "PGS" software.

### 2.2 Identification of transcripts differentially expressed

An initial ANOVA (ANalysis Of VAriance) model has been set up to identify differentially expressed transcripts between "Normal" condition and "Tumour" condition (practically a T-test, in which a unique variable was present). Differentially expressed transcripts were identified combining two distinct filtering criteria: Fold Change (FC), based on the amount of differential expression measured between "Normal" condition (N) and "Tumour" condition (T), and p-value used as a measure of the statistical reproducibility of the observed differential expression. To compensate for the high number of statistical tests run by the software and enhance the validity of the tests, a False Discovery Rate (FDR) correction has been applied to all statistical tests run. It should be noted that the FDR correction is indeed very stringent, meaning that the differentially expressed transcripts identified by different queries are likely to be validated by additional methods, i.e. qPCR. Moreover, to accomplish with the very high number of differentially expressed transcripts identified, a number of queries were run using more and more stringent FC cut-off values, going from 1.5 up to 3 fold. The search for differentially expressed transcripts between "Normal" condition and "Tumour" condition provided a very high number of targets, regardless of the highly stringent FDR correction applied. (Bolstad, B.M., Irizarry R. A., Astrand, M., & Speed, T.P. (2003) "A Comparison of Normalization Methods for High Density Oligonucleotide Array Data Based on Bias and Variance" Bioinformatics 19(2):185-193*;* Irizarry, R.A., Bolstad, B.M., Collin, F., Cope, L.M., Hobbs, B., Terence, P., & Speed, T.P. (2003) "Summaries of Affymetrix GeneChip probe level data" Nucleic Acids Research 31(4):15*;* and Irizarry, R.A., Hobbs, B., Collin, F., Beazer-Barclay, Y.D., Antonellis, K.J., Scherf, U., & Speed, T.P. (2002) "Exploration, Normalization, and Summaries of High Density Oligonucleotide Array Probe Level Data*"*).

### Results

Differentially expressed transcripts were identified combining two distinct filtering criteria: Fold Change, based on the amount of differential expression measured between "Normal" (N) and "Tumour" (T) conditions, and p-value used as a measure of the statistical reproducibility of the observed differential expression. MAL2 gene was identified with a very high fold change (T vs. N); see data reported below (as obtained by using the software "Partek Genomics Suite" 6.6):

| Gene Symbol | RefSeq | p-value(Condition) | p-value(Tvs.N) | Ratio(T vs. N) | Fold-Change(Tvs.N) | Fold-Change(Tvs. N) (Description) | F(Condition) | S(Condition) | SS(Error) | F(Error) |
|---|---|---|---|---|---|---|---|---|---|---|
| MAL2 | NM_052886 | 6,88E-08 | 6,88E-08 | 25,327 | 25,327 | Tup vs N | 349,972 | 54,3498 | 1,24238 | 1 |

### EXAMPLE 3 - Determination of MAL2 gene expression in CCSC lines by RT-PCR

Unregulated or overexpressed MAL2 can drive neoplastic transformation, tumour growth and survival. Therefore, the expression and regulation of MAL2 in human CCSCs were studied.

### 3.1 RNA extraction and cDNA preparation

Total RNA was extracted from: CCSC lines isolated from different patients with diagnosis of colon cancer, HNCSC lines isolated from normal mucosa tissues (taken from the above-mentioned patients) and "Colo 320" cell line (a commercially available human adenocarcinoma cell line) by using the "RNeasy Mini kit" (Qiagen).

### 3.2 Real Time PCR

Total RNA was isolated using TRIzol reagent (Invitrogen). 1 µg of total RNA was used for cDNA synthesis in 21 µl of volume reaction. 1 µl of cDNA was amplified in 25 µl of volume reaction. cDNA was obtained by using Superscript RNase H-Reverse Transcriptase (Gibco) by following the instructions provided by the manufacturer. 1 µg of total RNA was primed with oligo-dT for cDNA synthesis. The cDNAs were each individually amplified with the PCR primers of MAL2 ("Hs_MAL2_1_SG QuantiTect Primer Assay" manufactured by Qiagen). All cDNAs used as templates were previously normalized throughout a GAPDH RT- PCR using the following primer pair:
Forward (5'-CGGAGTCAACGGATTTGGTCGTAT-3')
Reverse (5'-AGCCTTCTCCATGGTGGTGAAGAC-3')

### Results

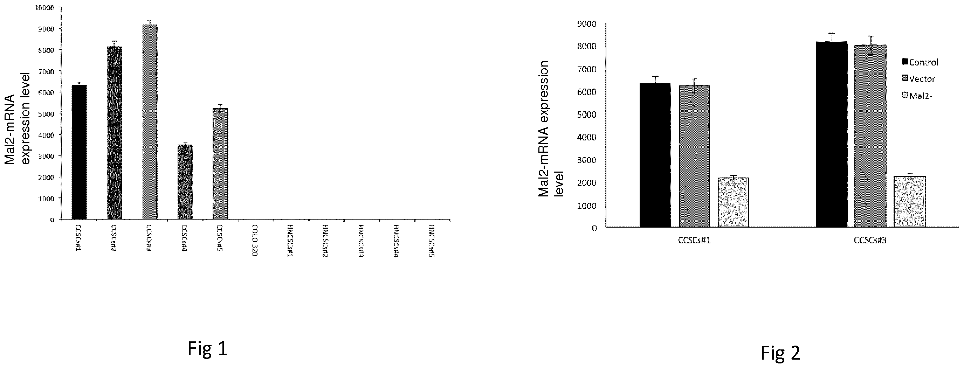

The over-expression of MAL2 gene in CCSC cells might reflect its malignant behaviour and its down-regulation might counteract tumourigenicity. As shown in Figure 1, MAL2 is widely upregulated in CCSC lines (n=5) as opposed to HNCSCs. Figure 2 shows the down regulation of expression of MAL2 gene in CCSC lines. The histogram shows means ± s.e.m. of 3 separate assays.

### EXAMPLE 4 - Experimental down-regulation of the MAL2 gene expression for determining its contribution to the regulation of the overall functional properties of CCSCs

### 4.1 CCSCs transfection

CCSC cells were transfected with a "Sure Silencing shRNA Plasmid" kit for MAL2 (Qiagen) by electroporation using a "Nucleofector™ Device" (Lonza AG) according to the proposed protocol. The kit includes 4 gene-specific shRNA plasmids (pGeneClip™) and one negative control plasmid. Each plasmid carries a shRNA (namely, a DNA sequence serving as template for transcription as shRNA) under the control of the U1 promoter and the PAC (puromycin resistance) gene. Briefly, 1 x 10⁶ CCSC cells were pelleted and resuspended in 100 µl of nucleofector solution ("Mouse NSC Nucleofector Kit" Lonza AG) together with 4µg of plasmid. The cells were then placed in an electroporation cuvette. Immediately after electroporation, 500 µl of pre-warmed medium was added to the cuvette and the cells were transferred into a culture flask containing pre-warmed CCSCs medium, and incubated at 37°C in a 5% CO₂ and 5% O₂ atmosphere. 24 hrs post transfection the medium was changed and made selective by adding 500 µg/ml of puromycin (Sigma) to generate stably transfected cell lines that allow a long term stable expression, defined and reproducible. The down-regulation of MAL2 was confirmed by RT-PCR assaying mRNA expression levels as previously described. The self-renewal index after the down-regulation of MAL2 on human CCSCs was assessed through clonogenic assays and growth kinetic.

### 4.2 Culture cloning

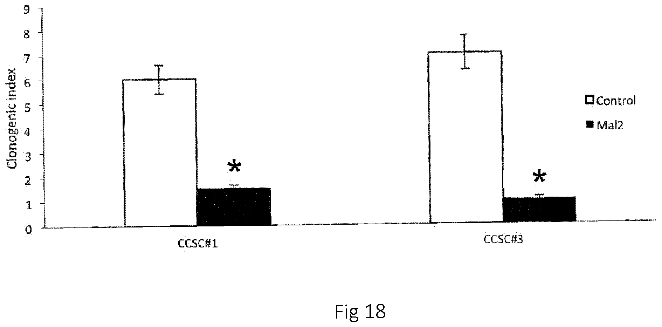

For the purpose of performing clonogenic assays, a pure single-cell suspension derived from the dissociation of human CCSCs and CCSCs-MAL2 was plated in single wells by an automated fluorescence-activated cell sorter (FACS) and grown as colon-spheres. The number of secondary spheres generated (clonal efficiency) was assessed after 7-10 days in vitro (DIV). The histogram graph of Figure 18 shows the results of the clonogenic assays: the columns correspond to the mean of 3 separate assays and the bars (at the top of the columns) correspond to s.e.m.

### 4.3 Growth curves

For the purpose of analysing the proliferation index of CCSCs and CCSC-MAL2 cells, colon-spheres suspensions were transferred to 15 ml tubes (Beckton Dickinson), centrifuged at 1000 rpm for 10 minutes and mechanically dissociated to a single-cell suspension. Cells were counted by Trypan blue exclusion and 200.000 cells/cm² viable cells were plated. The total cell number obtained at the following sub-culturing step (DIV) was plotted. At each subculture passage (every 3 days) a single-cell suspension was obtained and the total number of viable cells was counted by Trypan blue exclusion. 200.000 viable cells/cm² were re-plated under the same conditions. This led to the definition of kinetic parameters that provided indications of the role of the down-regulation of MAL2 gene in determining COLON-CSCs expansion rate (Gritti A., Parati E., Cova L., Frolichsthal P., Galli R., Wanke E., Faravelli L., Morassutti D., Roisen F., Nickel D.: "Multipotential stem cells from the adult mouse brain proliferate and self-renew in response to basic fibroblast growth factor", 1996 J NEUROSCI, vol. 16, pages 1091 - 1100, XP000881158).

### Results

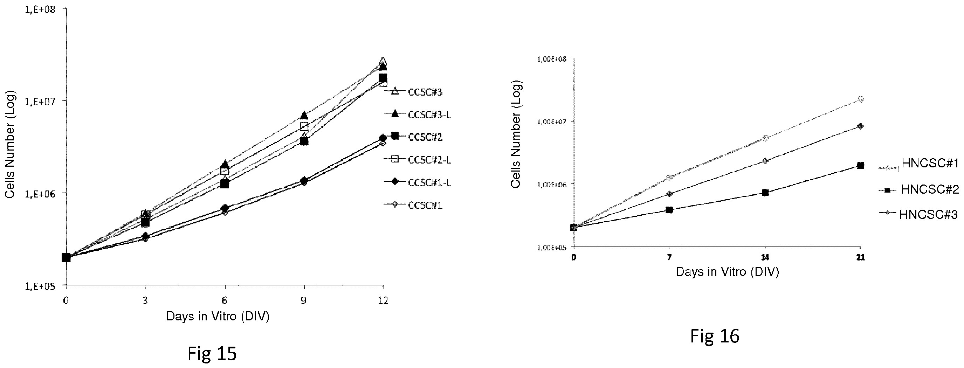

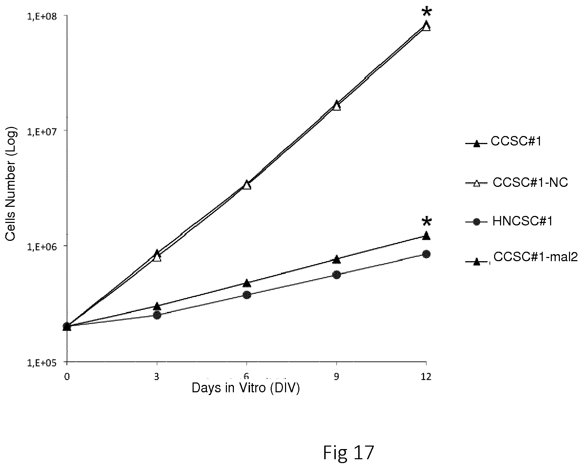

As shown in Fig 15 (graph showing the growth kinetics curves of 3 different colon cancer stem cells lines at early and late passages before the down-regulation of MAL2), both early and late passages of CCSCs revealed an inherent capacity for extensive, long-term exponential growth similar to the colon stem cells (see graph in Fig 16, showing the growth kinetics curves of human normal colon stem cells in culture), which property is suggestive of self-renewal. Figures 17 to 18 shows that the down-regulation of MAL2 gene on CCSCs affects the overall symmetry of division and the related self-renewal activity in human CCSCs. The growth differences in the growth kinetics were detected *in vitro* between CCSCs and CCSC-MAL2 cells with the former characterized by faster growth rate and the latter comprising slowly-dividing CCSCs. Particularly evident is the graph of Figure 17, showing 4 growth curves. Two of the curves are representative of corresponding CCSCs lines, one is representative of a CCSC-MAL2 line and one is representative of a HNCSCs line. It is undisputable that both the CCSC-MAL2 line and the HNCSCs line are characterized by an analogous growth rate, which is slower than the growth rate exhibited by the other two CCSCs lines. It can thus be stated that the down-regulation of MAL2 reduces the proliferation of colon cancer stem cells and depletes the colon tumour-initiating cell population *in vitro.* In this view, the effects of the enforced down-regulation of expression of MAL2 gene on tumourigenic properties in CCSCs were determined, as disclosed in the following Example 5.

### EXAMPLE 5 - Effects of MAL2 down-regulation on human CCSCs subcutaneous (s.c.) tumour xenograft growth

It has been assessed whether the *in vitro* reduction of the pool of tumourigenic CCSCs is related to a similar decline in the ability of CCSC-MAL2 cells to form tumours *in vivo,* in subcutaneous (s.c.) xenografts. In order to monitor and quantify tumour progression and treatment efficacy, non-invasive BLI (Bioluminescent Imaging) was used.

### 5.1 Transfection of CCSCs with reporter gene firefly luciferase

CCSCs and CCSC-MAL2 cells were transfected with the pGL4.51 (*luc*2/CMV/Neo) plasmid (Promega), which is a vector encoding the luciferase reporter gene *luc2* of *Photinus pyralis* L. (firefly), and selected with 500 µg of Neomycin. The efficiency of the transfection was assessed by *in vivo* Lumina analysis (Xenogen). Bioluminescent cells were serially diluted from 5000 to 100 cells in complete medium into black, clear bottomed "Nunc™96-Well" plates. D-Luciferin included in "ONE-Glo™ Luciferase Assay System" (Promega) was added 1:1(v/v) to each well (containing single cells resuspended in 100 µl of complete medium) 3 minutes before imaging. Imaging time was of 1 min/plate. The cells with the highest bioluminescent light emission were selected for a subcutaneous tumour xenografts growth (see paragraph 5.2 below) and these cells did not show changes in either growth rate or morphology in comparison with the parental cells (data not shown).

### 5.2 Subcutaneous tumour xenografts growth

The luc-CCSCs and luc-CCSC-MAL2 cells of paragraph 5.1 above were used for establishing tumour xenografts. 2 x 10⁶ luc-CCSC cells or 2 x 10⁶ luc-CCSC-MAL2 cells, in 100 µl of PBS mixed with an equal volume of Cultrex (Trevigen), were injected into the left flank of 3 groups of 10 immunocompromised athymic nu/nu 3-4 weeks old female mice (Charles River) using a 23-gauge needle (i.e. a needle having a nominal outer diameter of about 0.6 mm). Mice were monitored for 7-30 days (data not shown). Primary tumours were excised at the end of the experiment (i.e. 30 days after injection) and the excised tumours were analysed by *ex vivo* BLI.

### Results

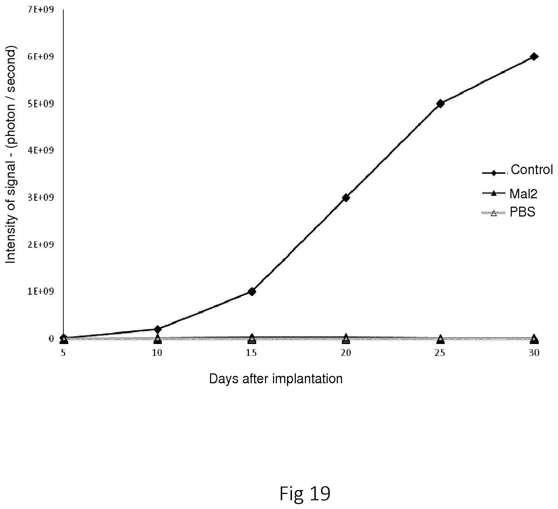

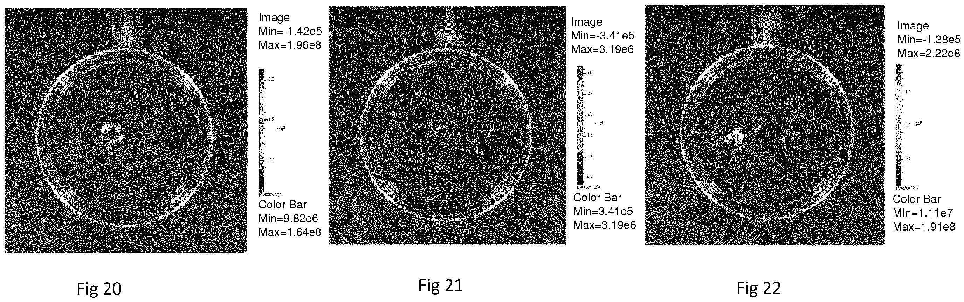

As clearly shown in Figures 19 to 22, down-regulation of MAL2 in COLON-CCSCs strongly inhibited tumourigenic ability of human CCSCs and tumour progression in a subcutaneous murine model. This produced a major drop of their growth *in vivo,* as clearly shown in the graph of Figure 19, which shows mean ± s.e.m. in the 3 groups of 10 mice and in which the changes of the bioluminescent signal over time accurately reflect tumour growth or regression. Since bioluminescence is a function of the number of metabolically active tumour cells rather than a volumetric measure of the tumour mass, its ability to solely measure viable cells offers a great advantage and a more accurate value of tumour physiology over traditional tumour mass volumetric measurements, which also include the contribution of dead or necrotic regions within the tumour. This allowed to determine whether the down-regulation of MAL2 could lessen and/or abolish tumourigenicity in CCSCs, as can be seen clearly in Figures 20 to 21. Figures 20 and 21 show the *ex vivo* BLI (Bioluminescence Imaging) images of two subcutaneous tumours of control (positioned onto two Petri dishes), which were induced in two corresponding mice respectively by a subcutaneous injection of luc-CCSCs and a subcutaneous injection of luc-CCSCs-MAL2. Both the tumours were excised after 30 days. Figure 22 show the comparison between the subcutaneous tumour of Figure 20 and the subcutaneous tumour of Figure 21. Both the tumours are positioned onto a same Petri dish (the luc-CCSCs-induced tumour is on the left and luc-CCSCs-MAL2-induced tumour is on the right) and the reduced light emission from luc-CCSCs-MAL2-induced tumour (on the right) confirms the effect of MAL2 gene down-regulation on CCSCs. While subcutaneous implantation is a popular and valuable method for modelling tumour cell growth, differences between the microenvironment of the skin and colon can cause rather dramatic differences in cell behaviour. The major limitations are that the implanted cells do not recapitulate many of the essential features of tumour growth in patients, rarely metastasize and the ectopic microenvironment greatly differs from that in the colon.

### EXAMPLE 6 - Evaluation of the efficacy of down-regulation of expression or activity of MAL2 on tumour growth through the use of a clinically orthotopic tumour model

### 6.1 Preparation of an animal (mouse) tumour model

2 x 10⁶ stable CCSC cells or CCSC-MAL2 cells transfected with pGL4.51 (firefly luciferase) plasmid (see Example 5, paragraph 5.1, above) were pelleted and resuspended with 30 µl of PBS with a syringe (Hamilton) having a 30-gauge (0,3 x 13 mm) needle (Beckton Dickinson). The transfected cells were subsequently transplanted into the wall submucosa of the ascending colon of nu/nu mice, according to the following procedure. Transplanted mice were anesthetized with 3% isoflurane in an induction box and, during subsequent surgery, with 2% isoflurane via mask. The abdomens of the anesthetized mice were disinfected with a solution of 70% ethylic alcohol and povidone (polyvinylpyrrolidone)-iodine. The skin of the mice was opened with toothed forceps and a scalpel, the abdominal wall was cut open and care was taken not to damage the abdominal organs. The caecum was identified and brought out of the abdomen, then the colon was identified. The caecum was put back into the abdomen and the colon was rehydrated with a warm saline solution. Then the transfected cells were injected in the ascending colon. The colon was rehydrated with the warm saline solution and put back into the abdomen. The abdominal wall was closed using a Vicryl 4-0 suture (continuous suture) and the skin closed with staples. A proper implantation into the ascending colon was confirmed 48 hrs-post injection by a localized and unique bioluminescent signal into the mice abdominal cavity. Mice successfully injected were imaged by BLI once a week from ventral views. 6.2 Evaluation of tumour growth and metastasis through Bioluminescence Imaging (BLI) - Analysis *in vivo* of tumour progression

The formation of the primary tumour and the subsequent metastasis was indirectly calculated once a week by sequential images taken with the "Xenogen - IVIS Lumina" imaging system (Caliper Life Sciences). According to the manufacturer's instructions for *in vivo* imaging, at the indicated times, mice were injected intraperitoneally (i.p.) with 250 µl of a solution of 15mg/ml D-Luciferin in PBS (Caliper Life Sciences). 3-5 minutes after injection, mice were anesthetized with 2% isoflurane using "Xenogen XGI-8 Anesthesia System" (Caliper Life Science) (Liu H., et al. (2010) "Cancer stem cells from human breast tumors are involved in spontaneous metastases in orthotopic mouse models" PNAS 107:18115-18120*).* A Luciferin kinetic study had been performed for this animal model to determine peak signal time: bioluminescence images were acquired 15 min after D-Luciferin injection using the "Xenogen - IVIS Lumina" imaging system. Imaging times ranged from 0.1 s to 1 min in automatic mode, depending on the tumour growth and time point. Luminescent measures were performed once a week, starting at the end of the first week after cells injection until the sixth or seventh week after cells injection. The low levels of light emitted from the bioluminescent tumours were detected by the "IVISR Camera System", integrated, digitalized and displayed. Regions of interest (ROI) from displayed images were identified around the tumour sites and were quantified as total flux (photons/s) using "Living Image" software (Xenogen). The light emitted from the bioluminescent cells was detected, digitalized and electronically displayed as a pseudo-colour overlay onto a grey scale animal image. Regions of interest (ROI) from displayed images were drawn automatically around the bioluminescent signals and quantified as photons/second (ph/s). Data were expressed as total photon flux (photon/second) and were analysed using "Living Image 3.0" software (Xenogen - Caliper Life Sciences). The experiment with control mice (mice transplanted with luc-CCSCs) was terminated on day 42, due the health conditions of the animals. The experiment was extended to 49 days with the mice transplanted with luc-CCSC-MAL2 cells, but there was no further bioluminescence signal due to the tumour regression.

### Results

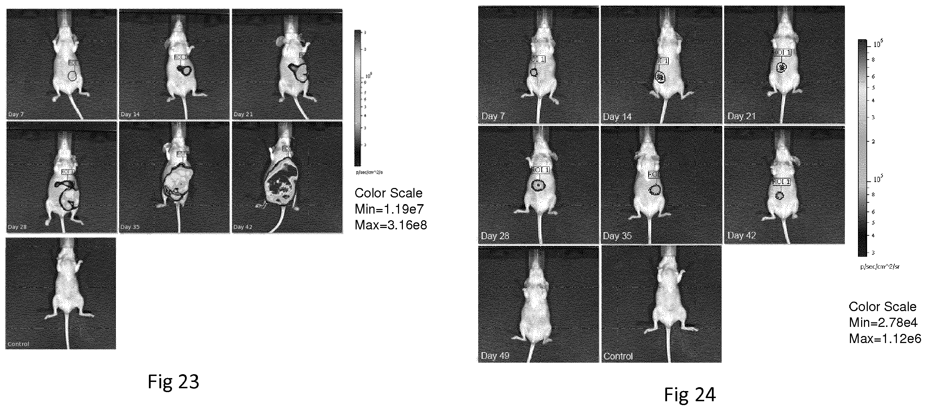

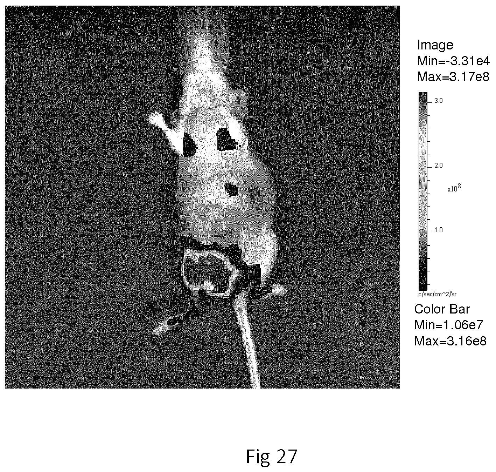

The formation of the primary tumour and the subsequent metastasis in mice transplanted with luc-CCSC-MAL2 cells was indirectly calculated once a week by sequential images (as disclosed in paragraph 6.2 above) and compared with those of the control animals, i.e. the mice transplanted with luc-CCSCs. These results indicate that the light emission from constitutively expressed luc gene is proportional to tumour cell burden and changes of the bioluminescent signal over time accurately reflect tumour growth or regression. Each of Figures 23 and 24 shows selected ventral view images from a corresponding representative mouse over time. The images from each corresponding view were set at the same pseudo-colour scale to show relative bioluminescent changes along time. Figure 24 shows sequential images of the *in vivo* bioluminescence monitoring of tumour progression and metastatic dissemination 7 weeks after transplantation of luc-CCSCs-MAL2, namely at 7, 14, 21, 28, 35, 42, 49 days (and control) after transplantation. Figure 24 clearly confirms that the *in vitro* down-regulation of MAL2 in luc-CCSC cells dramatically reduced tumour-initiating ability at the primary site of the transplantation (no primary tumour cells were detected at the point of injection) and inhibited the spread of the tumour metastasis to the various organs (no evidence of metastasis was identified). Figure 23 shows the monitoring of tumour progression and metastatic dissemination 7 weeks after transplantation of luc-CCSCs, namely at 7, 14, 21, 28, 35, 42 days (and control) after transplantation. It is clear that the bioluminescence monitoring shown in Figure 23 shows a corresponding signal increase from the growing and disseminating tumours. Figure 27 shows an *in vivo* ventral view of a representative mouse transplanted with luc-CCSCs. Spontaneous metastasis is evident in lymph nodes and lungs of this mouse, which confirms lymphatic and haematogenous dissemination.

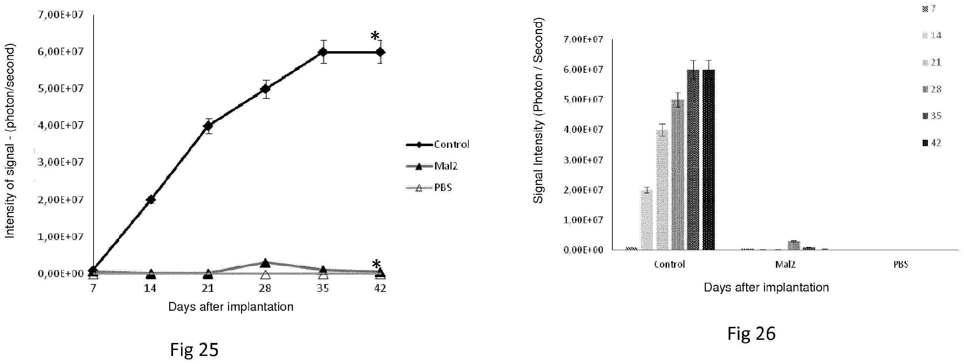

Figures 25 (broken line graph) and 26 (histogram graph) show the growth rate of primary tumours and regional metastasis as quantified weekly by bioluminescent intensity (total flux expressed in photons per second, p/s). The data in the graphs are mean ± s.d. of 3 different experiments (*P<0,001, Student's t test), involving the above-mentioned (see Example 5, paragraph 5.2, above) 3 groups of 10 mice. A region of interest (ROI) for each animal (n = 25) was defined at every time point. PBS was used as control-vehicle. After 6-7 weeks mice were sacrificed. Sacrificed animals undergone trans-cardiac perfusion/fixation with 100 ml of 0.15M NaCl followed by 250 ml of 0.1 M potassium phosphate buffer (KBS) containing 4% PFA. The trans-cardiac perfusion/fixation was carried out at a pressure of 120 mmHg, using a Watson-Marlow peristaltic pump. Tissue sections taken from xenografts were fixed in 4% paraformaldehyde for 48 hours and then transferred to 70% ethanol. Once fixed, the tissue samples were processed using Tissue-Tek VIP and embedded in paraffin. The blocks were sectioned at 4µm thickness on a Microm HM 200 cryotome, and stained with haematoxylin-and-eosin (H.E.) as per standard histopathological technique. Samples were viewed and photographed with a "Zeiss Axiophot-2" fluorescence microscope and "Leica DMIRE2" confocal microscope. No labelling was ever observed in control experiments when primary antibodies or antisera were omitted or, alternatively, when normal non immune serum was used.

### EXAMPLE 7 - Tumourigenic assay of metastatic cancer cells

### 7.1 Immunohistochemistry on tissue sections

Immunohistochemistry was performed on formalin-fixed paraffin-embedded tissue. Paraffin sections were dewaxed in xylene and rehydrated with distilled water. The slides were subsequently incubated with the following antibodies: pre-diluted Anti-CDX2 (Ventana Medical Systems Inc., Tucson AZ, USA,) and diluted 1:100 Anti-CK20 (Spring Bioscience, Pleasanton, CA, USA).

### Results

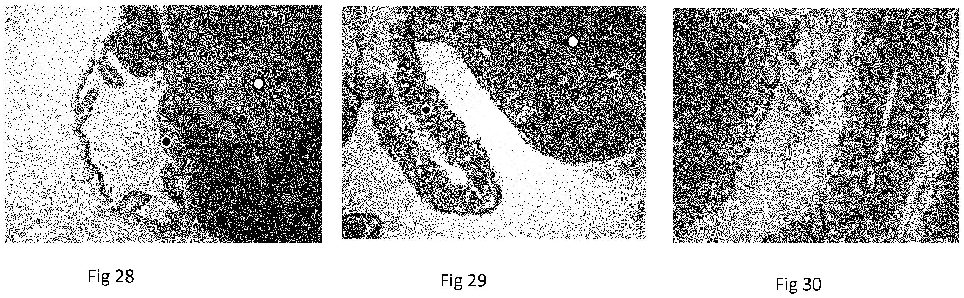

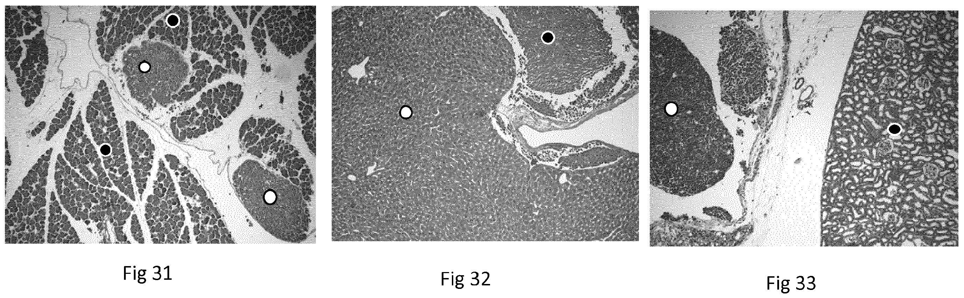

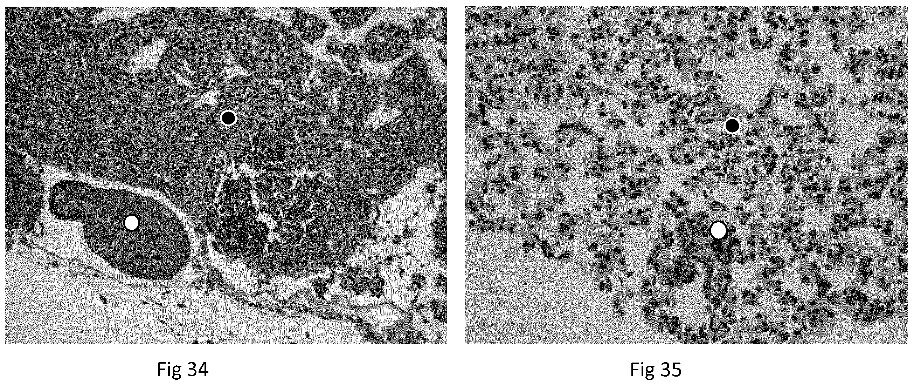

In order to confirm the presence of neoplastic cells, all the organs of the mice transplanted with luc-CCSC-MAL2 cells were investigated for the presence of neoplastic cells. No trace was found of neoplastic cells or metastasis development (data not shown) and, at the local primary site (Figure 30), there was no tumour development, which evidence confirmed the results observed with the BLI. On the contrary, as shown in Figures 28 and 29 (in which white spots indicate tumour cells and black spots indicate normal tissue), the mice transplanted with luc-CCSCs showed clear evidence of tumour progression and metastatic dissemination (metastatic tumours at 42 days after orthotopic implantation). The histology of the metastatic tumours, as highlighted by H.E. staining, revealed comparable staining patterns in both metastatic tissues and primary tumour cell locations. This is clearly shown in Figure 31 (mouse pancreas), Figure 32 (mouse liver), Figure 33 (mouse kidney), Figure 34 (mouse lymph node) and Figure 35 (mouse lung), in which white spots indicate tumour cells and black spots indicate normal tissue.

### EXAMPLE 8 - Analysis of the expression of CDX2 and CK20 in metastasis induced by intra-colon implantation of luc-CCSCs or luc-CCSCs-MAL2

The assessment of the properties of the CCSCs, both from primary tumours and from metastasis, was completed by analysing the expression of CDX2 and CK20 in the metastasis that were established in various organs by intra-colon implantation of luc-COLON-CSCs or luc-CCSCs-MAL2. The CDX2 (caudal type homeobox 2) gene is a transcription factor of the caudal homeobox family and is considered a good marker of primary and metastatic colorectal adenocarcinoma (Mazziotta R.M., et al. (2005) "CDX2 immunostaining as a gastrointestinal marker" Appl. Immunohistochem. Mol. Morphol. 13:55-60*).* The CK20 (cytokeratin 20) gene belongs to the epithelial subgroup of the intermediate filament family which, due to its restricted range of expression in humans, has been adopted as one of the markers for delineating the origin of metastasis of human adenocarcinomas from unknown primary sources (Wildi S., et al. (1999) "Characterization of Cytokeratin 20 expression in pancreatic and colorectal cancer" Clinical Cancer Res. 5: 2840-2847*).*

### Results

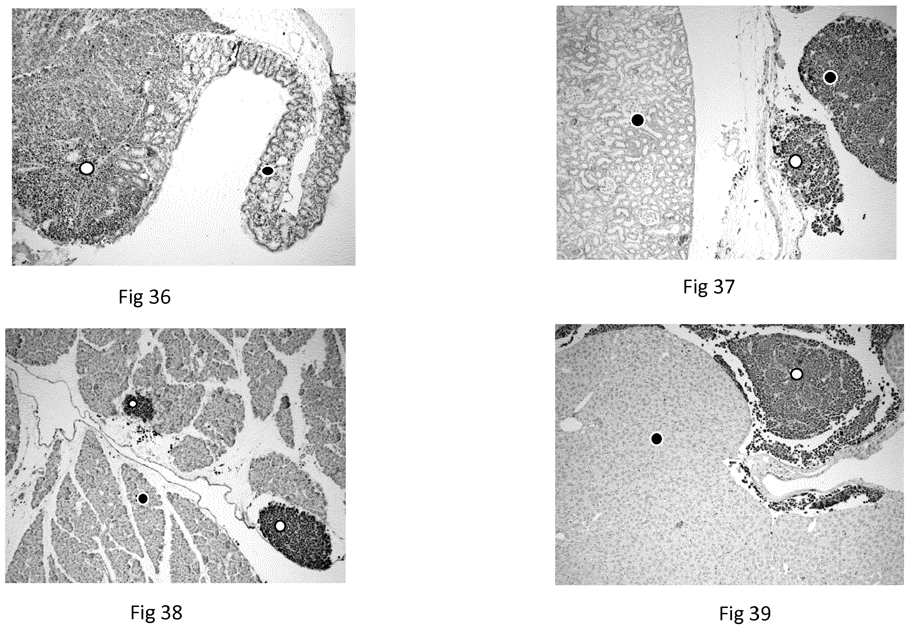

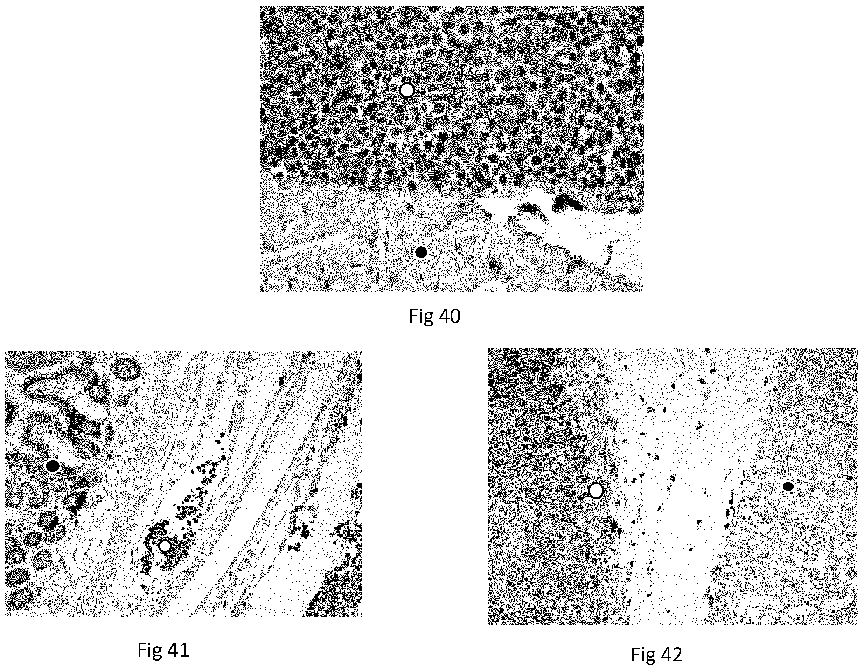

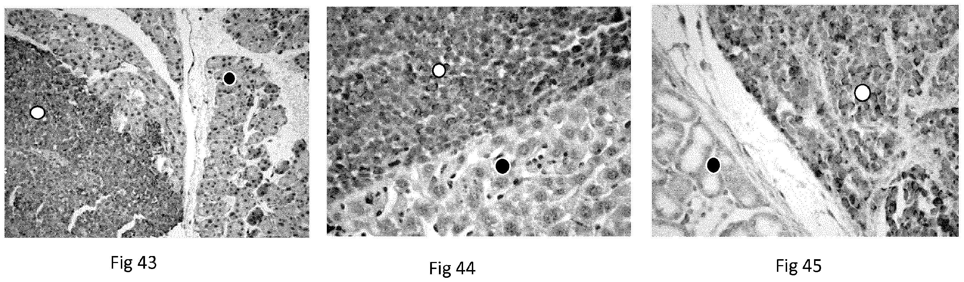

Immunostaining against CDX2 and CK20 markers revealed strikingly comparable staining patterns in both the primary and metastatic tumours from both cell types (luc-CCSCs and luc-CCSC-MAL2 cells), which showed features of poorly differentiated colon adenocarcinoma. This is clearly attested by: Figures 36 to 42, showing comparable staining patterns, as revealed by CDX2 immunohistochemical marker, in primary and secondary metastatic xenografts (white spots indicate tumour cells and black spots indicate normal tissue) respectively in mouse colon, kidney, pancreas, liver, muscle, small intestine and kidney; Figures 43 to 45, showing comparable staining patterns, as revealed by avidin-biotin-peroxidase complex and CK20 immunohistochemical marker, in primary and secondary metastatic xenografts (white spots indicate tumour cells and black spots indicate normal tissue) respectively in mouse pancreas, liver and kidney.

### EXAMPLE 9 - Comparison of features between cells of metastatic lesions and CCSCs from primary tumour

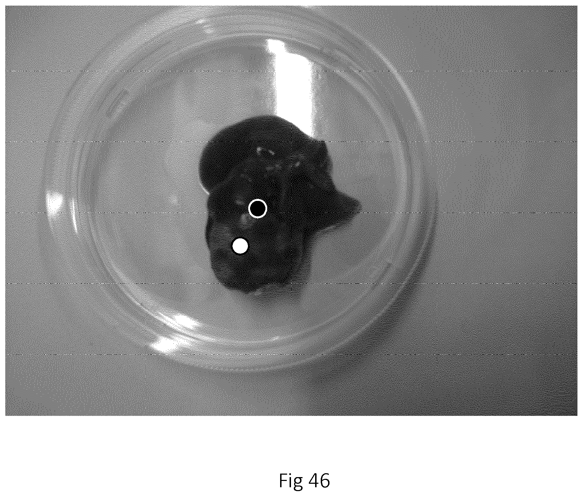

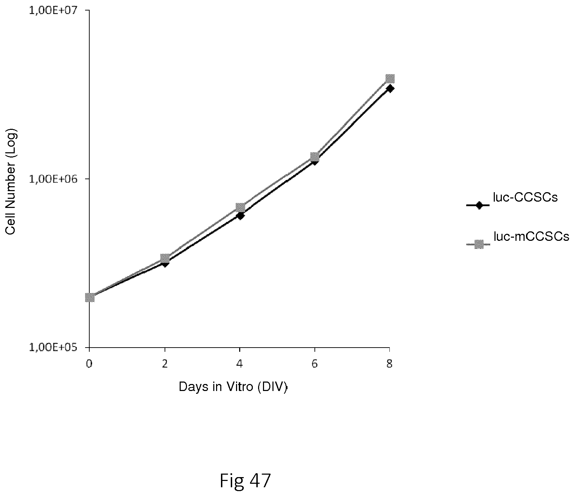

In order to investigate whether the metastatic lesions contained cells retaining cancer stem cell features that are similar to those of the CCSCs of the primary tumour, from which the metastatic lesions originated, cells were isolated from liver metastatic lesions. In particular, the liver shown in Figure 46 (positioned in a Petri dish) was used, which liver was excised from a nu/nu mouse orthotopically transplanted with luc-CCSCs (the white spots indicate the tumour cells and the black spots indicate the normal tissue). The cell were cultured as undifferentiated spheroids, as originally done for CCSCs of the primary tumour. It was found that the cells isolated from the liver metastasis could be maintained in an exponential growth state, significantly reproducing the exponential growth of the original CCSCs that were used to establish the primary tumour in the colon wall. This is clearly shown by the graph of Figure 47, illustrating the results of one experiment (representative of 3 experiments) of *in vitro* cell growth of primary luc-CCSCs and secondary (i.e. metastatic) luc-CCSs. Analysis of the tumourigenic capacity of the cells isolated and expanded from the liver metastasis was conducted by both subcutaneous and orthotopic implantation into nu/nu mice (data not shown).

### Results

Xenografts generated from secondary (metastatic) liver cancer cells resemble the original primary (colon) tumour and retained all of the features expected from true colon tumour-initiating cells. The down regulation of MAL2 gene inhibits, or however affects, the "culprit" of colon cancer, namely colon cancer stem cells (CCCSs) or colon tumour-initiating cells.

## Claims

1. Therapeutic medium, comprising an inhibitor of expression and/or activity of MAL2 gene, for use in the treatment of cancer.

2. Therapeutic medium according to claim 1, wherein said inhibitor of expression and/or activity of MAL2 gene is able to induce a down-regulation of said expression and/or activity.

3. Therapeutic medium according to claim 1, or 2, wherein said inhibitor of expression and/or activity of MAL2 gene comprises a shRNA.

4. Therapeutic medium according to any one of claims 1 to 3, wherein said inhibitor of expression and/or activity of MAL2 acts upon a nucleotide target-sequence that is comprised in the MAL2 gene.

5. Therapeutic medium according to claim 4, wherein said nucleotide target-sequence is selected from the group consisting of the nucleotide sequences of SEQ ID NO: 1, SEQ ID NO: 2, SEQ ID NO: 3 and SEQ ID NO: 4.

6. Therapeutic medium according to any one of claims 1 to 5, wherein the form of said therapeutic medium is selected from a group consisting of: a pharmaceutical composition, a lentiviral targeting vector and a liposome-nucleic acid particle.

7. Therapeutic medium according to claim 6, wherein said inhibitor of expression and/or activity of MAL2 gene is comprised in said pharmaceutical composition.

8. Therapeutic medium according to claim 6, wherein said inhibitor of expression and/or activity of MAL2 gene is comprised in said lentiviral targeting vector.

9. Therapeutic medium according to claim 6, wherein said inhibitor of expression and/or activity of MAL2 gene is comprised in said liposome-nucleic acid particle.

10. Therapeutic medium according to any one of claims 1 to 9, wherein said cancer is a colorectal cancer.


SEQUENCE LISTING

<110>  RHEMASTEM SRLS

<120>  Therapeutic medium for the treatment of cancer

<130>  488

<160>  4

<170>  PatentIn version 3.5

<210>  1
<211>  21
<212>  DNA
<213>  MAL2 gene of Homo sapiens_positions 215 to 235


<220>
<221>  exon
<222>  (1)..(21)

<400>  1
tgg ctc aaa ttg atg cta act                                          21
Trp Leu Lys Leu Met Leu Thr
1               5


<210>  2
<211>  21
<212>  DNA
<213>  MAL2 gene of Homo sapiens_positions 258 to 279


<220>
<221>  exon
<222>  (1)..(21)

<400>  2
ttg gtt gcc tcc tcc aat gtt                                          21
Leu Val Ala Ser Ser Asn Val
1               5


<210>  3
<211>  21
<212>  DNA
<213>  MAL2 gene of Homo sapiens_positions 374 to 394


<220>
<221>  exon
<222>  (1)..(21)

<400>  3
ctc ctg agt gat aac cag tat                                          21
Leu Leu Ser Asp Asn Gln Tyr
1               5


<210>  4
<211>  21
<212>  DNA

<213>  MAL2 gene of Homo sapiens_positions 519 to 540

<220>
<221>  exon
<222>  (1)..(21)

<400>  4
ctt cgt ctg cct gga gat tct                                    21
Leu Arg Leu Pro Gly Asp Ser
1               5